# EUROPEAN PATENT APPLICATION

(11) **EP 2 868 320 A1**
(43) Date of publication of application: **06.05.2015**
(21) Application number: 13191319.6
(22) Date of filing: 03.11.2013
(51) Int. Cl.: A61K 31/56, A61P 35/00, A61P 25/14

(54) **A composition or group of compositions for the treatment of human cells**

(71) Applicant: Flamina Holding AG, 6304 Zug (CH)
(72) Inventor: May, Michael, CH-6304 Zug (CH)
(74) Representative: Rutz & Partner

(57) **Abstract**

The invention relates to a composition for the treatment of the organelles of genetically healthy cells of human beings, particularly for preventive treatment of the cells supporting control system, comprising at least one active agent having strong competitively binding hormone antagonist properties combined with at least a first and/or a second carrier medium supporting the transport of molecules of said active agent through the cell-wall into the cytosol and organelles of cells. In preferred forms of applications, groups of at least a first and a second inventive composition are provided that comprise different carrier media and that are applicable in different parts of the body for forwarding the active agent via different pathways to a determined application zone.

## Description

The present invention relates to a composition, a group of compositions and a treatment that allow preventing dysfunctions of intracellular organelles of genetically healthy human cells.

### BACKGROUND OF THE INVENTION

The health of a human cell is directly linked to the health of the organelles located in that cell, such as the Nucleus, the Rough and Smooth Endoplasmic Reticulum, the Golgi Apparatus, and the Mitochondria. Only healthy organelles are able to deliver the required function, e.g. to assemble the required hormones, enzymes, coenzymes and other necessary proteins, at the right amounts, at the right times and at the right locations within the cell. These enzymes and co-products are of vital importance for intracellular repairs as for example DNA repair.

During a lifetime of a human being, every single gene is likely to have undergone a mutation on about 10¹⁰ separate occasions (see [1], Bruce Alberts, et al., Molecular Biology of THE CELL, New York 2002, 4th edition, page 1317). With an averaged lifetime of 70 Years and at 30,000 genes per cell, each cell must control and eventually repair about 100,000 damages per second. Detection, location and repair of each such likely damage, require many highly specialized enzymes, complex infrastructures, signal transmission pathways, many of them not yet known at all or fully understood. Only recently the discovery of over 4 million intrachromosomal and interchromosomal communications in the comparatively simple yeast cell, the discovery of about 200 switches per gene and the well-known but unexplainable fact, that the cell selects and produces the right proteins (about 30.000 proteins out of about 20³⁰⁰ different possible polypeptide chains), documents the enormous complexity of the cell (see [1], page 141).

The well-known fact, that one single catalase macro-molecule converts each second up to 40 million molecules of harmful hydrogen peroxide to water and oxygen, thereby preventing oxidative damage of important metabolic processes, underlines this vital importance of enzymes and their co products.

The fact is often neglected that all cellular enzymes are the product of many carefully organized processes that take place between the various cellular organelles.

Incorrectly repaired or unrepaired gene mutations, once established, can accumulate thus developing reactions and consequences that are typically detected only after a longer time period, e.g. of about one year. The unrepaired damages can propagate to neighbouring cells and can cause various cellular disorders that cause health problems.

Due to the complexity of the human cell, a complex pattern of malfunctions and diseases can occur that relate to the individual elements of the cell, namely the organelles.

For example, observations made during the past decades have suggested that mitochondrial malfunctioning is implicated in the pathogenesis of a variety of human disorders, including cancer and multiple neurodegenerative diseases, such as Parkinson's disease (PD), Alzheimer's disease (AD), amyotrophic lateral sclerosis (ALS), and Huntington's disease (HD). For a long time, mitochondria were known mainly as cellular 'power plants', although it soon became clear that their role in cellular physiology is not restricted to ATP production for metabolic demands. Hence, mitochondria were shown to be crucial for the regulation of intracellular Ca²⁺ homeostasis, especially under pathological conditions; they also produce reactive oxygen species (ROS), as well as reactive nitrogen species (RNS), which are involved in the regulation of various physiological processes, but might be harmful to the cell if produced excessively. In addition, mitochondria are key participants in several cell death pathways. All these functions are of critical importance for cell viability and physiology, and it is obvious that perturbation of mitochondrial functions might play an important role in the pathogenesis of various human diseases (see [2], Vladimir Gogvadze, Sten Orrenius, Boris Zhivotovsky; Mitochondria as targets for chemotherapy; Published online: Springer Science+Business Media, LLC 2009).

However not only key functions of the organelles themselves but also the interrelations and interdependencies between the organelles are highly important and can be the origin of a multifunctional disease. These interrelations and interdependencies, which are used to accomplish the many complicated biochemical and biomechanical processes, necessary for assembling each of the hundreds of different enzymes, co-products and related signal pathways, required for the maintenance of health, have not been explored sufficiently by now.

It would therefore be desirable to provide a composition and a group of compositions that enhance function and prevent dysfunctions of the organelles of cells in order to avoid irreversible cell deteriorations.

It would be desirable in particular to provide a composition and a method that allow routine treatment at an early stage of abnormal occurrence of cell deteriorations with a high impact on impaired cells while avoiding a degradation of the general condition of healthy cells and the well-being of a person under treatment.

More particularly it would be desirable to provide a composition and a group of compositions that allow preventive treatment of a person that is by age and or lifestyle predisposed to intra-cellular dysfunctions as, in particular, autocrine hormone production.

Still further, it would be desirable to provide a composition and a method that allow supporting normal cell-regulation processes, so to successfully retard physical and mental aging processes.

Moreover it would be desirable to provide the composition in a form that is easily and safely applicable individually.

An important object is further to reach the desired effect with a very low concentration of an active agent, so that side-effects are avoided.

### SUMMARY OF THE INVENTION

The above and other objects of the present invention are achieved by a composition according to claim 1 and a group of compositions according to claim 8.

The invention relates to a composition for the treatment of genetically healthy cells of a human being, particularly for preventive treatment of dysfunctions of organelle of cells and for supporting its control and repair system, comprising at least one active agent having pronounced competitively binding hormone antagonist properties combined with at least a first and/or a second carrier medium supporting the transport of molecules of said active agent into the cytosol of cells.

A preferred group of competitively binding progesterone antagonists are those of the formula below 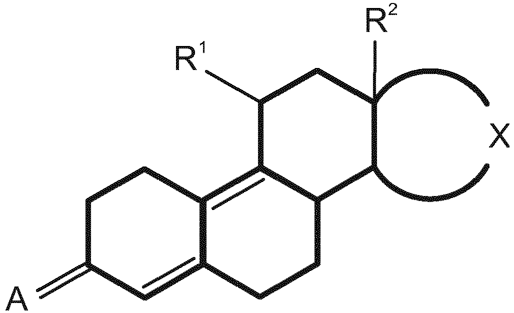 and derivatives thereof that are able to inhibit progesterone activities. Such compounds are described for example in [3], US4634695A, or [4], US8053426, which are fully enclosed herewith by reference.

The at least one active agent is a competitively binding progesterone antagonist such as Aglepristone (CAS-Number: 124478-60-0, ATC-Code: QG03XB90), Mifepristone (CAS-Number: 84371-65-3, ATC-Code: G03XB01) or a competitively binding Human Chorionic Gonadotropin-(HCG) antagonist or a combination thereof.

Preferably the first carrier medium is a cell penetrating peptide, a permease, a carrier protein, a polar aprotic solvent such as Dimethyl sulfoxide (DMSO), an alcohol, a lipid, a liposome, or a mixture thereof, suitable for transporting (the) molecules of said active agent into the cytosol and the organelles, particularly the Endoplasmic-Reticulum, the Golgi apparatus, the Mitochondria and the Nucleus of the cells.

The second carrier medium is preferably
a) a nutrition article, such as a nutritional supplement dedicated to health-improvement or delivery of energy in high concentration, wherein the second carrier medium comprises 0.1 to 3 mg/100 g of said active agent; or
b) a support for physiological and pharmaceutical effective application, such as a tablet or capsule, that comprises 0.5 to 5 weight per cent per total weight w%/W, preferably 1 to 3 w%/W, of said active agent; or
c) a suppository base such as Glycerine or Bisacodyl that is contained in an implantable suppository and that comprises 0.5 to 6 w%/W, preferably 1 to 3 W%/W, of said active agent; or
d) a solution, suspension, or emulsion that is prepared for inhalation by the user and that comprises 0.5 to 6 w%/W, preferably 1 to 3 w%/W, of said active agent; or
e) a cream, a lotion, a gel, a paste, or a plaster, that is prepared for transdermal delivery of the active agent, which is administered with a dosage of 4 to 12 w%/W, preferably 6 to 10 w%/W.

Selection of an appropriate second carrier medium allows application of the active agent conveniently and efficiently to the field of desired applications. Integrating the active agent in energy bars that are consumed before sportive activities are undertaken is most efficient for the transfer of the active agent.

A person predisposed to intracellular dysfunctions can counteract the same with very small amounts of the active agent. Even with the intermittent use of the inventive application of the compound (composition), such person can significantly reduce the risk of pathological cell deteriorations.

In further applications, units e.g. in form of a tablet, capsule, drops for oral or preferably sublingual use are provided, comprising at least one of the first and/or second carrier medium and the active agent with a daily dose in the range of 0,5 mg to 3 mg, preferably 1 mg to 2 mg/100 gr. Such application units can be prescribed for intermittent use. For people over 50 Years of age it is advisable to absorb about twice this amount of the active compound.

In order to increase the impact of the active agent on the organelles, the second carrier medium can be composed by multivitamin products, as are well known and sold in pharmacies, preferably combined with trace elements, such as Selenium, Zink, Phosphor and Copper.

In order to further support intra-cellular functions, regulation and optimal enzyme production, the composition further comprises at least
a) one agent for causing reduction of endogenous glycation, such as Carnosine, Luteolin, Benfontiamine, Pyridoxal-5'-phosphate, or coenzyme Q10; and/or
b) one antioxidant and components stimulating intracellular functions, such as L-cysteine, Resveratrol, Quercetin-dihydrate, DHEA, Green-tea, Curcumin extracts from Gynostemma pentaphyllum, or Cinnamon; and/or
c) one agent supporting enhanced ATP production, such as phyto-therapeutic substances as Shilajit, Vinblastine, Saw-palmetto extract, Artimisia-annua, Methyl-jasmonate, Ascorbic-acid, Ginseng and the like.

In a situation where a person is already subject to a beginning epithelial disease, transporting the active agent towards a specific field of application along a single pathway may cause undesirably high local concentrations of the active agent, which is avoided by using at least two different pathways, along which the active agent is transported to the field of application.

Therefore according to a preferred application of the invention, a group of at least a first inventive composition and a second inventive composition is provided that comprise different carrier media (and) that are applicable in different parts of the body for forwarding the active agent via different pathways to a common application field or target cell complex. Hereby the first composition that is provided with a first concentration of the active agent and the second composition that is provided with a second concentration of the active agent are adapted to one another in such a way that the desired amount of active agent is delivered to the target cell complex and the concentration of the active agent along a specific pathway does not exceed a defined level, or can be increased for treatment purposes, if advisable or necessary.

In a preferred form of application, the first composition is provided for transdermal application and the second composition is provided for inhalatory application or oral application, if a problem was detected in the lungs for instance.

In another form of application, the first composition is provided for transdermal application and the second composition is provided for use via a suppository.

For the prevention or treatment of intracellular irregularities, the active compound with a first carrier is applicable in any medically accepted way. For younger persons, a subcutaneous implant with a predetermined release rate of 50 to 100 mg active agent per Year is a stress-free solution.

It is recommended to administer the inventive compound in an age-related manner, preferably taking further into account personal strengths and weaknesses of each person. In particular, the rate and dosage of the application of the inventive composition is increased with the age of the user. In the event that a person is pre-disposed to intracellular deteriorations, the rate of application or dosage is adapted accordingly.

Details of the invention have been described above. Further examples as well as details and explanations of inventive forms of applications are provided below.

Our internal preliminary tests have proven that the inventive compositions provide excellent results also in cases that have gone beyond the preventive phase. The application of the inventive compounds assists and restores intracellular repair processes and thereby also impedes propagation of eventual or established dysfunctions into adjacent or remote cells.

If self-repair cannot be accomplished by the cell itself, it is equally important to assist and enhance the multitude of intracellular biomechanics necessary for apoptosis, avoiding thereby progression towards malignancy. With the application of the inventive compounds, even this delicate task of controlling and intensifying apoptosis can be fulfilled, supporting a multi-cell complex for returning to a desired healthy functional state.

### DETAILED DESCRIPTION OF THE PREFERRED FORMS OF APPLICATIONS

The inventive composition or group of compositions, for the treatment of organelles of genetically healthy cells of a human being, particularly for preventive treatment of the cell and for supporting its control systems, comprise a pharmacologically active and physiologically acceptable and safe amount of an active agent, which has strong competitively binding hormone-antagonist properties.

The active agent is combined with a carrier medium that enhances the transport of the molecules of said active agent into the cytosol and organelles, particularly into the Endoplasmic-Reticulum, the Golgi Apparatus, the Nucleus and the Mitochondria, of the cells or targeted cell complex.

With the preventive measures, the start and uncontrolled deterioration of the efficiency of the enzyme production and an eventual reduction of enzyme efficiency in cells can successfully be avoided or counteracted. Self-regulation of the cells, cell signalling and apoptosis, is supported so that the state of the cell functions remains within a non-pathological bandwidth.

The carrier medium selected for transporting the molecules of said active agent through tissues and through the cell-membranes into the cytosol of the cells can be a cell penetrating peptide, a permease, a carrier protein, preferably a polar aprotic solvent as Dimethyl-sulfoxide (DMSO), an alcohol, a lipid, a liposome, a mixture thereof, or the like.

The claimed compounds are also suited for the treatment of disorders in epithelial cells. Particularly for topical treatment at a determined epithelial area, the combination of the active agent of the compound with a medium to enhance penetration through epithelial tissue and cell membranes, thereby accelerating and improving the preventive and/or treatment processes is strongly increasing the effect of the active agent.

Prevention of pathological cell activities can be obtained with small quantities of the applied active agent. While the invention is acting pre-emptive in order to prevent pathological activities, it is understood that cell activities that have gone beyond the non-pathological bandwidths can be brought back to a normal state by applying the inventive compounds. However, in such an event, depending on the state of the malfunction and the age of the individual, the dosage of the active compound and the number of individual compounds applied and their dosage with the active agent are adapted accordingly. Therefore it will regularly be possible to correct pathological cell activities, if action is taken early, that is, before cells develop drug resistance.

An advantageous treatment of epithelial cell defects is obtained, if two or more inventive compositions are administered concomitantly; topically and parenterally. Thereby the inventive treatment occurs simultaneously from the inner side, i.e. the region of the blood vessels, and from the outer or topical side. Particularly positive results have been obtained by this inventive 'bilateral' application of the active agent after a comparably short duration of the applications.

For the application onto an afflicted epithelial area, a supporting carrier or medium for the inventive compound is preferably adapted or optimized accordingly. For example, for the treatment of epithelial problems in the colon, the carrier may be a suppository. For skin problems, creams, lotions, gels, oils, sprays, patches, and the like, adapted for topical or transdermal application, are the preferred choice. For the treatment of old age Type 1 Diabetes or bucal-cavity problems, oral or sublingual application is an alternative, and for problems in the lung, a spray to be inhaled is a good carrier for the active agent.

For gastro-intestinal problems, the composition is preferably administered in a (XR) slow release formula.

For preventive applications, the amount of the active agent is preferably administered in an age and severity related manner, according to the following examples.

For preventive use and persons less than 50 years of age, 50-200 mg of the active agent, or about 1 to 2 mg per Kg per year in several sub portions are sufficient. For a person over 50 years: 200-500 mg per year, partitioned into 2 or more time intervals, preferably via drug eluting stents, transdermal patches, included within multivitamin compounds or as drops for sublingual use are advisable. Very good results have also been obtained by application with a cream, oil, a spray or a gel on surfaces very close to blood vessels like, for example mucosae in our internal preliminary testing.

For treatment purposes, higher dosages and applications, according to the type and location of the problem, 30 to 100 mg, preferably 10 to 50 mg per day for adult persons are recommendable during the treatment.

The compositions are principally applicable by conventional routes of administration, as for example topically, transdermally, orally, and so on. Therefore the active agent can be combined (to) with a variety of basically known admixtures, as for example creams, oils, lotions, trans-dermal patches, suppositories, aerosols, gels, or subcutaneous implants for slow release, comparable to contraceptive implants, subcutaneous pellets and the like. For a parenteral application, also sublingual or administrations by injection are considered.

The amount of the active agent in these combinations for preventive purposes is preferably kept at 0.5% to the 5%, preferably 1% to 3% by weight or about 20 mg per gr. of carriers.

In order to further support cell regulation and regeneration, the composition further comprises preferably
a) one agent for causing reduction of endogenous glycation, such as Carnosine, Luteolin, Benfontiamine, Pyridoxal-5'-phosphate, or coenzyme Q10; and/or
b) one antioxidant and components stimulating optimised functioning of the organelle, in particular in respect of enzyme production, such as L-cysteine, Resveratrol, Quercetin-dihydrate, DHEA, Green-tea, Curcumin extracts from Gynostemma pentaphyllum, or Cinnamon; and/or
c) one agent supporting enhanced ATP production, such as phyto-therapeutic substances as Shilajit, Vinblastine, Saw-palmetto extract, Artimisia-annua, Methyl-jasmonate, Ascorbic-acid, Ginseng and the like.

Compositions for preventive treatment comprise 0.1% to 5%, preferably 0.5% to 3% by weight of said active agent and are preferably admixed into health-sustaining compounds or mixtures of multivitamins and minerals as multivitamin tablets, effervescent tablets and the like.

Particularly for oral or sublingual application, the compositions are preferably combined with other health sustaining compounds as multivitamin tablets, with or without minerals, which in many applications can serve also as pharmacologically acceptable fillers.

Preferably, antioxydants, supporting ATP production in the mitochondria and/or supporting enzyme production, are added to the basic composition. Of particular interest are antioxidants as Resveratrol, Quercetin-dihydrate, DHEA, Green-tea, Curcumin, et cetera.

### References

[1] Bruce Alberts, et al., Molecular Biology of THE CELL, New York 2002, 4th edition;
[2] Vladimir Gogvadze, Sten Orrenius, Boris Zhivotovsky; Mitochondria as targets for chemotherapy; Published online: Springer Science+Business Media, LLC 2009);
[3] US4634695A
[4] US8053426

## Claims

1. A composition for the treatment of organelles and their related signal pathways of cells of a human being, particularly treatment for preventing dysfunctions and for supporting the cell's control systems, comprising at least one active agent having strong competitively binding hormone antagonist properties combined with at least a first and/or a second carrier medium for supporting the transport of molecules of said active agent through the cell-wall of the related cells.

2. Composition according to claim 1, whereby the at least one active agent is a competitively binding progesterone antagonist such as Aglepristone or a competitively binding Human Chorionic Gonadotropin-(HCG) antagonist, improvements thereof or a combinations thereof.

3. Composition according to claim 1 or 2, whereby the first carrier medium is a cell penetrating peptide, a permease, a carrier protein, a polar aprotic solvent such as Dimethyl sulfoxide (DMSO), an alcohol, a lipid, a liposome, or a mixture thereof suitable for transporting the molecules of said active agent into the cytosol and the organelles, particularly the endoplasmic-reticulum, the Golgi apparatus, the mitochondria and the nucleus of the cells.

4. Composition according to one of the claims 1 to 3, whereby the second carrier medium is
a) a nutrition article, or a nutritional supplement dedicated to health-improvement or delivery of energy in high concentration, wherein the second carrier medium contains 0.5 to 3 mg/ 100 g, preferably 1 to 2 mg/100 grams or about 1 mg/100 gr of said active agent; or
b) a support for physiological and pharmaceutical effective application, such as a tablet, capsule, cream, paste, emulsion, lotion, oil, suppository, spray, lozenge, gel, plaster for transdermal delivery that comprises 0.5 to 6 w%/W preferably 1 to 3 w%/W, particularly 2 w%/W of said active agent; or
c) a solution, suspension, or emulsion that is prepared for inhalation by the user and that comprises 0.5 to 6 w%/W, preferably 1 to 3 w%/W, of said active agent; or
d) a cream, a lotion, a gel, a paste, or a plaster, that is prepared for transdermal delivery of the active agent, which is administered with a dosage of 0.5 to 6 w%/W, preferably 1 to 3 w%/W.

5. Composition according to claim 1 to 4, whereby the daily treatment dose of the active agent during treatment purposes is in the range of 5 mg to 100 mg, preferably 40 mg to 60 mg, and is enclosed in a unit consisting at least of the first and/or second carrier medium.

6. Composition according to claim 1 to 3, whereby the active agent is admixed into second carrier medium comprising vitamin or multivitamin products, preferably combined with trace elements, such as Selenium, Zink, Phosphor and Copper.

7. Composition according to one of the claims 1 to 6, further comprising at least,
a) at least one agent for causing reduction of endogenous glycation, such as Carnosine, Luteolin, Benfontiamine, Pyridoxal-5'-phosphate, or coenzyme Q10; and/or
b) at least one antioxidant and components stimulating intracellular functioning, such as L-cysteine, Resveratrol, Quercetin-dihydrate, DHEA, Green-tea, Curcumin extracts from Gynostemma pentaphyllum, or Cinnamon; and/or
c) at least one agent supporting enhanced ATP production, such as phyto-therapeutic substances as Shilajit, Vinblastine, Saw-palmetto extract, Artimisia-annua, Methyl-jasmonate, Ascorbic-acid, Ginseng or the like.

8. Group of at least a first and a second composition according to one of the claims 1 to 7 that comprise different carrier media and that are applicable in different parts of the body for forwarding the active agent via different pathways to a predetermined application zone, whereby the first composition that is provided with a first concentration of the active agent and the second composition that is provided with a second concentration of the active agent are adapted to one another in such a way that the desired amount of active agent is delivered to the field of application and the concentration of the active agent along a specific pathway does not exceed specific values.

9. Group of at least a first and a second composition according claim 8, wherein the first composition is provided for transdermal application and the second composition is provided for inhalatory application.

10. Group of at least a first and a second composition according claim 8, wherein the first composition is provided for transdermal application and the second composition is provided for oral application.

11. Group of at least a first and a second composition according claim 8, wherein the first composition is provided for oral or inhalatory application and the second composition is provided for transdermal application by means of a suppository or the like.

12. Group of at least a first and a second composition according claim 8, wherein for the treatment of cells at the origin of an epithelial disease, the first composition is provided via blood vessels (inner side) to one side of the epithelium, and the second composition is provided onto the skin or 'outer' side of the epithelium, preferably essentially at the same time.

13. Group of at least a first and a second composition according claim 8, wherein the first composition is provided via oral application and the second composition is provided via inhalatory application and a third composition is provided via implantation or within a suppository.
